# EUROPÄISCHE PATENTANMELDUNG

(11) **EP 2 937 074 A1**
(43) Veröffentlichungstag der Anmeldung: **28.10.2015**
(21) Anmeldenummer: 14001490.3
(22) Anmeldetag: 25.04.2014
(51) Int. Cl.: A61K 8/46, A61Q 19/00, A61K 31/10, A61P 17/02

(54) **Topisch anwendbare Zubereitung mit Methylsulfonylmethan zur Verbesserung der Vernarbung**

(71) Anmelder: Reinacher, Petra, 79111 Freiburg (DE)
(72) Erfinder: Reinacher, Petra, 79111 Freiburg (DE); Oesterling, Martin, 79238 Ehrenlürde (DE)
(74) Vertreter: Börjes-Pestalozza, Henrich

(57) **Zusammenfassung**

Topische Zubereitung, beinhaltend Methylsulfonylmethan (MSM), und weitere für topische Anwendungen übliche Zusätze, und ihre Verwendung zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen oder anderer Narbenmissbildungen. Die Zusammensetzung kann weiter vorzugsweise Hyaluronsäure oder deren Salze, und *Aloe,,* z.. *Aloe*-Blattsaft, *Aloe-vera*-Saft und/oder Aloe-Gel oder Extrakt, und gegebenenfalls weitere Zusätze für topische Zusammensetzungen beinhalten.

## Beschreibung

Die Erfindung betrifft topisch anwendbare Zubereitungen (Formulierungen) zur Behandlung und Pflege von Bindegewebsunregelmäßigkeiten der Haut und subkutan, wie Narben, deren Verwendung und deren Herstellung.

Eine Reihe von Creme- und Gelzubereitungen sind bekannt und werden für die Behandlung von Narben und anderen Bindegewebsbasierten Gewebsneubildungen eingesetzt. In der Regel beinhalten sie Silikone wie Dimethicon, daneben werden Antioxidantien, Polypeptide, Zwiebelextrakt (z.B. Scar Esthetique^{®} der Firma Scarheal Inc., Largo, Florida) oder komplexe Mischungen aus Lipiden und Pflanzenextrakten und Natrium-Hyaluronat (z.B. Revitol Scar Cream, siehe http://naturallabs.de/106,revitol-scar-cream-narben.html).

Dimethylsulfon, oft auch als Methylsulfonylmethan bezeichnet, wird als Nahrungsergänzungsmittel für Menschen und Tiere, beispielsweise für Pferde, verwendet. Es kommt in vielen tierischen und pflanzlichen Organismen vor und kommt so auch in die menschliche Nahrung. Es soll dazu beitragen, einen Mangel an organischem Schwefel im menschlichen und tierischen Organismus zu vermeiden. Vgl. z.B.: https://de.wikipedia.org/wiki/Dimethylsulfon.

Das US-Patent US 8,071,140 beschreibt eine Zusammensetzung, die Wasser, Glyzerin, Plantago major-Blattpulver oder -extrakt, Aloe babadensis-Blattsaft, Kakaobutter und Sheabutter beinhaltet, US 5,604,200 beschreibt Mischungen zur Wundtherapie, welche unter anderem Hyaluronsäure und Plasma-Fibronectin beinhalten.

Aus kosmetischen und teilweise auch psychologischen Gründen ist eine Behandlung von Narben mit dem Ziel, ihr Erscheinungsbild und die Gewebestruktur zu verbessern, erstrebenswert.

Indikationen sind beispielsweise Narben im Gesicht, etwa durch Akne oder Windpocken oder dergleichen, und/oder andere bindegewebsbedingte sichtbare Unregelmäßigkeiten, wie Dehnungsstreifen und verbrennungsbedingte sowie operationsbedingte Vernarbungen, die oft eine Behandlung mit dem Ziel der Verbesserung des Erscheinungsbildes und der Gewebestruktur erstrebenswert machen.

Während die genannten und weitere Angebote in dieser Richtung manches versprechen, besteht nach wie vor ein Bedürfnis nach einer guten Behandlungsmöglichkeit für derartige Gewebsunregelmäßigkeiten.

Es wurde nun überraschend gefunden, dass ein Zusatz von Methylsulfonylmethan (nachfolgend auch als MSM) bezeichnet) in topischen Zubereitungen eine im Erscheinungsbild verbesserte Narbenbildung, auch bei der Verhinderung von Narbenwucherungen und Narbenwülsten, bewirken kann und auch eine deutliche Verbesserung gegenüber unbehandelten Narben bewirken kann. Die Erfindung betrifft daher in einer ersten Ausführungsform eine topische Zubereitung, beinhaltend Methylsulfonylmethan, insbesondere zur Verwendung zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen, und weitere für topische Anwendungen übliche Zusätze.

In einer weiteren Ausführungsform betrifft die Erfindung die Verwendung von Methylsulfonylmethan zur Herstellung einer topischen Zubereitung zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen, unter Verwendung weiterer für topische Zubereitungen üblicher Zusätze.

Eine Verkörperung der Erfindung betrifft auch die Verwendung einer Methylsulfonylmethan und ein oder mehrere weitere für topische Anwendungen übliche Zusätze beinhaltenden topischen Zusammensetzung zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen.

Die nachfolgenden spezifischeren Definitionen dienen zum Beschreiben bevorzugter Varianten der mit ihnen beschriebenen Begriffe wobei in den Ausführungsformen/Verkörperungen der Erfindung ein, mehrere oder alle der Begriffe durch die spezifischeren Definitionen ersetzt werden können, was zu bevorzugten Ausführungsformen der Erfindungsgegenstände führt.

Die erfindungsgemäßen bzw. erfindungsgemäß verwendeten Formulierungen eignen sich (vorzugsweise nach bereits erfolgter Wundheilung) zur Behandlung von Vernarbungen, wie postoperativen oder von verletzungen herrührenden Narben oder von Verbrennungsnarben, wie auch zur Behandlung von Dehnungsstreifen (Striae cutis atrophicae oder Striae cutis distensae (auch Schwangerschaftsstreifen)).

Unter einer topischen Zusammensetzung ist in erster Linie eine solche zu verstehen, die (vorzugsweise zum einstweiligen Verbleib dort) auf die Haut (also zur kutanen Anwendung insbesondere narbige oder Dehnungsstreifen tragende Bereiche) aufgetragen werden kann, beispielsweise in Form einer Salbe, einer Creme, eines Gels, einer Paste, einer Lotion, eines Sprays, einer Lösung, einer Tinktur, eines Puders, eines wirkstoffhaltigen Pflasters oder dergleichen. Besonders bevorzugt sind hydrophile Zubereitungen, wie hydrophile oder wasseraufnehmende Salben, hydrophile Pasten, wasserbasierte Sprays oder insbesondere hydrophile Gele.

Neben Methylsulfonylmethan können bei allen Ausführungsformen der Erfindung weitere die Narbenerscheinung verbessernde Zusätze als Wirkstoffe vorgesehen sein, wie Hyaluronsäure oder deren Salze, beispielsweise ein Erdalkalimetallsalz, wie das Natriumsalz, und Aloe, insbesondere in Form von Aloe-Blattsaft (auch als "Echte Aloe", dem aus dem Blattsaft durch Eindampfen bis zur Trockene gewonnenen Produkt, Succus Aloes inspissatus), Aloe-vera-Saft- (z.B. 25-%-ig), -Öl oder -Gel oder Aloe-Extrakt, oder ferner Silikone wie Dimethicon.

Bezogen auf die Wirkstoffe liegt in erfindungsgemäßen bzw. erfindungsgemäß zu verwendenden topischen Zubereitungen MSM vorzugsweise in einem Anteil von 5 bis 100 Gew.-%, beispielsweise von 20 bis 80 Gew.-%, wie z.B. von 25 bis 50 Gew.-%, vor.

Bezogen auf die Wirkstoffe liegt vorzugsweise in erfindungsgemäßen bzw. erfindungsgemäß zu verwendenden topischen Zubereitungen Hyaluronsäure oder deren Salz ein einem Anteil von vorzugsweise in einem Anteil von 5 bis 100 Gew.-%, beispielsweise von 20 bis 80 Gew.-%, wie z.B. von 25 bis 50 Gew.-%, vor.

Bezogen auf die Gesamtmenge an Wirkstoffen können erfindungs-gemäße topische Formulierungen weitere übliche Zusätze beinhalten, wie Lösungsvermittler, beispielsweise Alkohole, wie Ethanol oder Isopropanol, beispielsweise, diesmal bezogen auf die Gesamtzubereitung (Gesamtformulierung) in einem Gewichtsanteil von 1 bis 50 Gew.-%, z.B. von 2 bis 10 Gew.-%, sowie Wasser, beispielsweise in einem Anteil von 50 bis 99 Gew.-%, z.B. von 80 bis 95 Gew.-%.

Daneben können ferner noch weitere die Bindegewebsbildung unterstützende Wirkstoffe vorliegen, wie Collagen, Proteine, Aminosäuren, Vitamine oder dergleichen, beispielsweise in einem Anteil an den Wirkstoffen von 0 bis insgesamt 20 Gew.-%.

Weitere Zusätze sind ferner möglich, wie Emulgatoren, z.B. Cetylstearylalkohol oder Carrageenan, Feuchthaltemittel, wie Glyzerin, Propylenglykol, Pentylenglykol oder Harnstoff, Macrogol-20-glyzerinmonostearat, Konservierungsmittel, wie Alkalimetallsorbate, z.B. Kaliumsorbat, pH-Stabilisatoren, wie Citronensäure, etherische Öle, wie α-Bisabolol, Gelbildner, wie Carbomer, Carmellose-Natrium, Celluloseether, wie Hydroxyethylzellulose, oder Gelatine oder Pektine, oder Mischungen von zwei oder mehr davon, beispielsweise in einem Anteil, bezogen auf die Gesamtzubereitung, von 0 bis 20 Gew.-%.

Wo "ferner" verwendet wird, ist darunter zu verstehen dass Merkmale/Bestandteile nach diesem Wort in bevorzugten Ausführungsformen der erfindungsgemäßen bzw. erfindungsgemäß zu verwendenden Zubereitungen nicht vorgesehen sind.

Die erfindungsgemäßen Zubereitungen können nach üblichen galenischen Verfahren unter Mischen der verwendeten Komponenten hergestellt werden.

Die erfindungsgemäßen Formulierungen werden in üblichen Behältnissen konfektioniert, beispielsweise in Tuben, Spraydosen, Tiegeln, Dosierspendern, Flaschen aus Glas oder Kunststoff, Kapseln oder (im Falle von Pflasterzubereitungen) transdermalen therapeutischen Systemen.

Die Anwendung der erfindungsgemäß verwendbaren bzw. erfindungsgemäßen Zubereitungen erfolgt topisch durch Aufbringen, wie Aufsprühen, Aufpinseln oder Aufreiben, vorzugsweise an den Orten der Haut, die zu behandelnde Unregelmäßigkeiten aufweisen, und ggf. ferner in unmittelbar darum liegenden Bereichen.

Die Erfindung betrifft auch die in den Ansprüchen, inbesondere den Unteransprüchen, und der Zusammenfassung beschriebenen Erfindungsgegenstände, weshalb die Ansprüche und die Zusammenfassung hier durch Bezugnahme aufgenommen werden.

Das nachfolgende Beispiel dient der Illustration der Erfindung, ohne ihren Umfang einzuschränken:

### Beispiel: Gel mit MSM, Hyaluronsäure und Aloe

Folgende Ingredientien wurden in den angegebenen Mengen miteinander vermischt und ergaben nach 24 Stunden Stehenlassen bei Raumtemperatur stabile Gele, die in Dosierspender abgefüllt wurden:
910 g Aqua Purificata
50 g Ethanol 90 %
10 g MSM
10 g Aloe-vera¹
10 g Natriumhyaluronat

### )¹ von Caesar & Loretz GmbH, Hilden, Deutschland

Das resultierende Gel ist transparent und fettet nicht, ist somit geeignet zum Auftragen auch an sichtbaren Körperstellen wie Gesicht, Hals und Décollete und kann auch an schwierigeren Stellen (Schulter, Finger, Gelenke) angewendet werden. Die Inhaltsstoffe versorgen die Haut mit Feuchtigkeit, ohne abzudecken oder zu fetten.

Die Anwendung des Gels erfolgt zweimal täglich, morgens und abends, über zwei Monate.

Es kann im Vergleich mit unbehandelten Probanden oder unbehandelten Narbenbereichen gezeigt werden, dass mit dem vorliegenden Gel behandelte Narbenbereiche ein besseres Erscheinungsbild als unbehandelte Bereich aufweisen. Es erfolgt die Einstellung eines Feuchtigkeitsgleichgewichts der behandelten Stellen, und die Wiederherstellung der verletzten Hautflächen wird unterstützt. Das Gel eignet sich zur Vorbeugung und Pflege von unschönen Narben nach Abschluss der Wundheilung , insbesondere von Narbenwucherungen und -wülsten. Es macht die Narben glatter, weicher und flacher, reduziert die mit Narben häufig einhergehenden Begleiterscheinungen wie Spannungsgefühl und Juckreiz und kann zum völligen Verblassen roter oder dunkler Narben beitragen bzw. führen.

## Patentansprüche

1. Topische Zubereitung, beinhaltend Methylsulfonylmethan (MSM), und weitere für topische Anwendungen übliche Zusätze.

2. Topische Zusammensetzung nach Anspruch 1 zur Verwendung zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen.

3. Topische Zusammensetzung nach einem der Ansprüche 1 oder 2, weiter beinhaltend Hyaluronsäure oder deren Salze, und Aloe, insbesondere in Form von Aloe-Blattsaft, Aloe-vera-Saft und/oder Aloe-Gel oder Extrakt, und gegebenenfalls weitere Zusätze für topische Zusammensetzungen.

4. Topische Zusammensetzung nach einem der Ansprüche 1 bis 3, **dadurch gekennzeichnet, dass** der Anteil an MSM bei 5 bis 100 Gew.-%, beispielsweise von 20 bis 80 Gew.-%, wie z.B. von 25 bis 50 Gew.-%, liegt.

5. Topische Zusammensetzung nach einem der Ansprüche 1 bis 4, **dadurch gekennzeichnet, dass** der Anteil an Hyaluronsäure oder deren Salz bei 5 bis 100 Gew.-%, beispielsweise von 20 bis 80 Gew.-%, wie z.B. von 25 bis 50 Gew.-%, liegt.

6. Topische Zusammensetzung nach einem der Ansprüche 1 bis 5, **dadurch gekennzeichnet, dass** es sich um ein Gel handelt.

7. Topische Zusammensetzung nach einem der Ansprüche 1 bis 6, bei der es sich um ein hydrophiles Gel handelt.

8. Topische Zzusammensetzung nach einem der Ansprüche 1 bis 7, **dadurch gekennzeichnet, dass** sie als weitere Zusätze einen Alkohol, insbesondere Ethanol, und Wasser beinhaltet.

9. Verwendung von Methylsulfonylmethan zur Herstellung einer topischen Zubereitung, wie in einem der Ansprüche 1 bis 8 beschrieben, zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen.

10. Verwendung einer Methylsulfonylmethan und ein oder mehrere weitere für topische Anwendungen übliche Zusätze beinhaltenden topischen Zubereeitung, wie in einem der Ansprüche 1 bis 8 definiert, zur kosmetischen oder vor allem therapeutischen Behandlung von unregelmäßigen Bindegewebsbildungen der Haut und der subkutanen Gewebe, wie insbesondere von Narbengewebe, verbrennungsbedingten Narben und Dehnungsstreifen.
